# EUROPÄISCHE PATENTANMELDUNG

(11) **EP 0 790 058 A1**
(43) Veröffentlichungstag der Anmeldung: **20.08.1997**
(21) Anmeldenummer: 97102244.7
(22) Anmeldetag: 13.02.1997
(51) Int. Cl.: A61K 31/485, A61K 9/70

(54) **Verwendung eines transdermalen therapeutischen Naloxon-Systems (Naloxon TTS)**

(30) Priorität: 15.02.1996 DE 19605590
(71) Anmelder: Inresa Arzneimittel GmbH, 79114 Freiburg (DE)
(72) Erfinder: Harrant, Gernot, Dipl.-Ing., 67000 Strasbourg (FR)

(57) **Zusammenfassung**

Die Erfindung betrifft die Verwendung eines transdermalen therapeutischen Systems (TTS) mit einem Gehalt an Naloxon für die Intoxikations-Behandlung bei ambulanter oder stationärer Opioid-Behandlung und/oder die Obstipations-Behandlung.

## Beschreibung

Die Erfindung betrifft die Verwendung eines transdermalen therapeutischen Systems (TTS) mit einem Gehalt an Naloxon.

Naloxon ist ein reiner Opioidantagonist und durch das Fehlen einer intrinsischen Opioidwirkung das Mittel der Wahl bei Opioidintoxikationen bzw. zur Aufhebung von Opioidwirkungen. Der Wirkstoff wird daher von der WHO als "Essential Drug" eingestuft.

Starke chronische Schmerzen, wie sie vor allem bei Tumorerkrankungen auftreten, erfordern eine wirksame analgetische Behandlung, und zwar auch mit Opiaten, die in einer Häufigkeit bis zu 80 % zu Verstopfungen führt. Diese ist mit den herkömmlichen Laxantien nicht immer zufriedenstellend zu behandeln.

Naloxon kann die Opioid-induzierte Obstipation aufheben, ohne die erwünschte Analgesie zu beeinflussen; vergleiche beispielsweise Sykes in The Lancet, 337 (1991) 1475 ff und Basiliko et al. in Digestive Diseases and Sciences, 32 (8) (1987) 829-832.

Oral verabreichtes Naloxon ist nur zu 3 bis 8 % bioverfügbar. Die Hauptmenge wird im Magen-Darm-Trakt oder in der Leber zu unwirksamen Abbauprodukten metabolisiert. Deshalb muß Naloxon oral beispielsweise in Form von Tabletten sehr hoch dosiert werden; vergleiche beispielsweise Kreck et al. in The Lancet, (1982) 261-262 und Kreck et al. in Gastroenterology, 86 (5 Part 2) (1984) 1144.

Außerdem ist eine orale Gabe deshalb unzweckmäßig, weil die Wirkung nur kurze Zeit anhält und die Einnahme mehrmals am Tage erfolgen müßte. Es bleibt daher bisher die parenterale Gabe, die jedoch der akuten Notfallbehandlung vorbehalten bleibt, und zwar bei Intoxikationserscheinungen nach Opioidgabe beispielsweise im Krankenhaus. Auch hier ist wegen der kurzen Wirkdauer von Naloxon (Eliminations-Halbwertzeit 1 bis 2 Stunden) die Injektion mehrfach in bestimmten Zeitabständen durchzuführen. Aus verständlichen Gründen ist eine parenterale Gabe bei der Obstipation älterer Menschen ungeeignet. Für beides besteht ein Bedarf an einer länger wirksamen Arzneiform.

Transdermale therapeutische Systeme (TTS) mit einem Gehalt an Naloxon sind bereits bekannt, wozu beispielsweise auf DE-A-3 606 892, DE-A-4 423 850 und EP-A-0 178 140 verwiesen wird. Dieser Stand der Technik gibt jedoch keine Lösung für die bei Opiat-Intoxikationen oder Obstipationen auftretenden Probleme.

Die der Erfindung zugrundeliegende Aufgabe wird nun durch die Verwendung eines transdermalen therapeutischen Systems (TTS) gelöst, das
- eine Trägerschicht,
- eine Matrix mit einem Gehalt an Naloxon als Wirkstoff, wobei die Matrix von der Trägerschicht getragen wird, und
- eine fakultative Abziehschicht umfaßt, und zwar für
- die Intoxikations-Behandlung bei ambulanter oder stationärer Opioid-Behandlung und/oder
- die Obstipations-Behandlung.

Ferner wird die der Erfindung zugrundeliegende Aufgabe durch die Verwendung eines transdermalen therapeutischen Systems (TTS) gelöst, das
- eine Trägerschicht,
- eine Matrix mit einem Gehalt an Naloxon als Wirkstoff, wobei die Matrix von der Trägerschicht getragen wird, und
- eine fakultative Abziehschicht umfaßt, und zwar
zur Herstellung eines in Verkehr bringbaren Präparats für
- die Intoxikations-Behandlung bei ambulanter oder stationärer Opioid-Behandlung und/oder
- die Obstipations-Behandlung.

Die Erfindung betrifft damit die Verwendung eines auf der Haut anzubringenden Medikaments, auch transdermales therapeutisches System (TTS) genannt, und zwar zur Verabreichung der Substanz Naloxon = 1-N-Allyl-7,8-dihydro-14-hydroxy-normorphinon an den Menschen.

Die Erfindung beruht auf der Feststellung, daß Naloxon aus einem transdermalen therapeutischen System (TTS) langsam und in kleiner Menge durch die Haut des Menschen in den systemischen Kreislauf gegeben werden kann. Damit ist die Möglichkeit gegeben, nach der initialen parenteralen Gabe von Naloxon bei Patienten, die durch eine Opiatgabe Intoxikationen aufweisen, eine langsam ausschleichende Naloxonzufuhr über mehrere Tage durchzuführen.

Erfindungsgemäß ist die genannte Arzneiform aber auch zur Behandlung der Obstipation gut geeignet, weil sie kontinuierlich die benötigten kleinen Mengen an Naloxon an den Körper abgibt. Es treten keine Plasmaspiegelpeaks auf, und da die Substanz nicht vor Eintritt in den Kreislauf die Leber passieren muß, erfolgt auch kein erheblicher Abbau wie nach oraler Gabe.

Die erfindungsgemäße Verwendung des genannten Medikaments hebt also die Wirkungen von Opioiden auf und auch die oft als Nebenwirkung auftretende Obstipation. Das Medikament antagonisiert nicht nur eine opioid-induzierte, sondern auch die sogenannte idiopatische Obstipation. Letztere ist besonders bei gereatrischen Menschen eine häufig vorkommende Erkrankung. Als Ursache dafür wird angenommen, daß bei den Menschen, die darunter leiden, ein Überschuß an endogenen Opioiden besteht.

Die Behandlung der chronischen Obstipation mit einem Naloxon enthaltenden TTS ist aber auch deshalb zu empfehlen, weil es sich hierbei um ein neues Therapieprinzip handelt. Bisher angewendete Laxantien, wie Anthrachinone, Aloe und Senna aus dem pflanzlichen Bereich, oder chemische Substanzen, wie Bisacodyl, sind besonders bei chronischer Anwendung entweder mit Nebenwirkungen verbunden oder führen zu Elektrolyt-Stoffwechselstörungen, was wiederum die Obstipation verstärkt. Naloxon jedoch besetzt bestimmte Opioid-Rezeptoren und bietet damit ein grundsätzlich anderes Wirkprinzip.

Bei der erfindungsgemäßen Verwendung kann das Naloxon als freies Naloxon, Naloxon-Hydrochlorid oder eines seiner verträglichen Naloxon-Salze vorliegen. So wird erfindungsgemäß unter Naloxon die Base und/oder das Hydrochlorid und/oder das Hydrochlorid mit Kristallwasser und/oder ein verträgliches Naloxonsalz verstanden.

Die erfindungsgemäße Verwendung kann mit einer selbstklebenden Matrix vorgenommen werden.

Es wird z.Z. untersucht, ob mit einem Naloxon enthaltenden TTS am Menschen Plasmaspiegel erzielt werden können, die für die hier beschriebenen Anwendungsbereiche erforderlich sind.

## Patentansprüche

1. Verwendung eines transdermalen therapeutischen Systems (TTS), umfassend
- eine Trägerschicht,
- eine Matrix mit einem Gehalt an Naloxon als Wirkstoff, wobei die Matrix von der Trägerschicht getragen wird, und
- eine fakultative Abziehschicht für
- die Intoxikations-Behandlung bei ambulanter oder stationärer Opioid-Behandlung und/oder
- die Obstipations-Behandlung.

2. Verwendung eines transdermalen therapeutischen Systems (TTS), umfassend
- eine Trägerschicht,
- eine Matrix mit einem Gehalt an Naloxon als Wirkstoff, wobei die Matrix von der Trägerschicht getragen wird, und
- eine fakultative Abziehschicht
zur Herstellung eines in Verkehr bringbaren Präparats für
- die Intoxikations-Behandlung bei ambulanter oder stationärer Opioid-Behandlung und/oder
- die Obstipations-Behandlung.

3. Verwendung nach Anspruch 1 oder 2, **gekennzeichnet** durch freies Naloxon, Naloxon-Hydrochlorid oder ein verträgliches Naloxon-Salz.

4. Verwendung nach einem der vorhergehenden Ansprüche, **gekennzeichnet** durch eine selbstklebende Matrix.
